# EUROPEAN PATENT SPECIFICATION

(11) **EP 3 900 658 B1**
(45) Date of publication and mention of the grant of the patent: **14.05.2025**
(21) Application number: 20749064.0
(22) Date of filing: 30.01.2020
(51) Int. Cl.: A61B 18/14, A61B 18/12, A61B 18/00, A61B 90/00

(54) **MULTIPOLAR ABLATION DEVICE**
MULTIPOLARE ABLATIONSVORRICHTUNG
DISPOSITIF D'ABLATION MULTIPOLAIRE

(30) Priority: 30.01.2019 CN 201910089932; 31.01.2019 CN 201910096490
(43) Date of publication of application: 27.10.2021
(73) Proprietor: SYMAP MEDICAL (SUZHOU) LTD, Suzhou, Jiangsu 215123 (CN)
(72) Inventor: WANG, Jie, Suzhou, Jiangsu 215123 (CN)
(74) Representative: PGA S.p.A., Milano, Succursale di Lugano
(86) International application number: PCT/CN2020/074075
(87) International publication number: WO 2020/156496

(56) References cited:
- EP-A2- 2 942 023
- WO-A1-2018/106939
- CN-A- 101 610 735
- CN-A- 107 374 725
- CN-A- 107 374 725
- CN-A- 109 793 568
- CN-U- 203 089 369
- US-A- 6 162 216
- US-A1- 2001 031 961
- US-A1- 2016 278 845
- US-A1- 2016 317 210
- US-A1- 2017 014 177
- US-B1- 9 119 628

## Description

### BACKGROUND

### Technical Field

The present invention belongs to the field of a minimally invasive medical apparatus, and particularly relates to a radio frequency generator for ablation and a multi-electrode ablation device for transmitting energy in the trachea and bronchus.

### Related Art

The chronic obstructive pulmonary disease is a progressive disease which may cause obstruction of the lung airways, restricting airflow from going in and out of the lungs, such as asthma, emphysema, and chronic obstructive pulmonary diseases. Therefore, patients with the chronic obstructive pulmonary disease have difficulty in breathing and have symptoms such as coughing, wheezing, shortness of breath, chest tightness, and mucus (asthma attacks), and need clinical treatment, a lot of medical resources are consumed, and hospitalization and life-threatening danger may be caused. The causes of the chronic obstructive pulmonary disease are as follows: airway smooth muscle contraction, secretion of too much mucus by the airway glands, thickening of the airway wall smooth muscles due to inflammation, and changes of anatomical structures of the tissues around the airway.

The pathological hyperplasia and excessive and inappropriate contraction of the airway smooth muscles in the lung airway wall of the patients is one of pathological mechanisms of the chronic obstructive pulmonary disease. Therefore, reducing or eliminating pathologically hyperplastic airway smooth muscles is an option for treating the chronic obstructive pulmonary disease.

At present, the method for treating the chronic obstructive pulmonary disease such as asthma, emphysema and the chronic obstructive pulmonary disease mainly used clinically is using medicine treatments such as adrenaline medicine, theophylline medicine and hormones, or sputum excretion, anti-inflammation and the like for symptomatic treatment, which requires long-term medication, and cannot cure this type of diseases. Moreover, some patients are still unable to effectively control their condition after using inhaled corticosteroids (ICS) and long-acting β receptor agonists (LABA).

An existing minimally invasive ablation technique can reduce the pathologically hyperplastic airway smooth muscles. During the implementation of this treatment, a catheter is positioned in the airway, and electrode arrays on the tail end of the catheter expand to contact with the airway wall. By moving the catheter, energy is gradually transmitted to multiple parts of the trachea to remove the pathologically hyperplastic airway smooth muscles.

The safety and effectiveness of an ablation apparatus used in bronchial radio frequency ablation based on the prior art have defects, for example, the wall attaching condition of the ablation electrodes cannot be monitored and displayed; and for another example, at the moment the ablation is started, the great radio frequency energy is applied, so that the temperature uprush is greater after a set temperature is reached, this kind of suddenly applied and (or) suddenly changed radio frequency energy has the stimulation on the respiratory tract of the patient, and the great temperature uprush has a threat to the safety of the patient. Additionally, in the treatment process of the bronchial radio frequency ablation, the temperature of ablation electrodes is affected by frequent and complicated disturbance due to the change of the airflow caused by the breathing movement of the patient, the sliding of the electrodes caused by the chest movement of the patient, and the change of the attachment degree caused by unstable grip of an operator, and the general proportional integral control algorithm is easy to generate oscillation and overshoot, and is difficult to adapt to these complicated external disturbances, so that the ablation treatment effect is interfered.

The ideal bronchial radio frequency ablation should avoid repeated ablation at the same site. However, due to the carelessness or misoperation of the operator or no reminding function provided in a used apparatus in the practical clinic operation, after once ablation is completed, and the ablation is started again without catheter (electrode) transferring or sufficient transferring amount, which may cause the repeated ablation at the same site, causing permanent and irreversible damage to the airway tissue, or even airway fistula. The present invention adopts the following control mechanisms: the logic relationship among the impedance, power and temperature is defined, the temperature of the site to be ablated is detected before each ablation is applied, if the temperature of the site to be ablated is higher than 40°C to 60°C, preferably 45°C, ablation is not started, and the like, so that a radio frequency generator for ablation of the present invention has a protection mechanism of preventing repeated ablation.

Document CN107374725A discloses a multipolar ablation device for transmitting energy in the trachea and bronchi.

Document US20170014177A1 discloses methods and a system for thermally-induced renal neuromodulation. Document US9119628 B1 relates to methods and devices for improved precision in finding one or more nerves and then interrupting the transmission of neural signals through the target nerve.

### SUMMARY

An objective of the present invention is to provide a safer and more effective device for transmitting energy in the trachea and bronchus aiming at the defects in the prior art.

A radio frequency generator according to the invention is disclosed in the appended claims 1-10.

A multi-electrode ablation device according to the invention is disclosed in the appended claims 11-15.

### Advantages of the present invention

(1) The present invention defines a logic relationship among the impedance, power and temperature, the generated and controlled direct current, alternating current and radio frequency energy are precisely controlled, the temperature, impedance or tension signal is collected, processed and displayed, and the ablation effectiveness is determined according to the change of the impedance or tension signal, and the change of the impedance is one or more of the falling value of impedance, the change rate of impedance, the change in the change rate of impedance, or the change of impedance from falling to rising. The radio frequency output power is adjusted by using the closed loop control system by a segmentation control method to control the ablation temperature, and the temperature dynamic smoothening is utilized to treat various kinds of disturbances. Therefore, the safety and the effectiveness of the system are further ensured, i.e., the conditions of wrong ablation or ablation incapability cannot occur, and the condition of repeated ablation or excessive ablation cannot occur.
(2) According to the present invention, attachment degrees of each treatable site of the bronchus and different electrode are determined by using a segmentation proportional integral control algorithm, and the bronchial radio frequency generator for ablation can control the radio frequency output power so that the temperature of ablation electrodes can reach the ablation temperature within 3 s, additionally, the temperature uprush after the ablation temperature is reached is less than 3°C, generally ranges from 0.5°C to 1.5°C. The temperature is stably maintained at the ablation temperature, and a fluctuation is smaller than 1°C, and is generally smaller than 0.5°C. In the whole ablation treatment process, the radio frequency output power smoothly changes without suddenly applied and (or) suddenly changed radio frequency energy.
(3) According to the present invention, the above temperature dynamic smoothening can adapt to various complicated disturbance, a radio frequency output power smoothly changes in the whole ablation treatment process, a sudden change radio frequency energy cannot occur, the temperature keeps stable, the fluctuation is very small, and oscillation and overshoot cannot occur even if very frequent and very violent disturbance occurs. Additionally, temperature uprush possibly caused by disturbance can be well inhibited, and the temperature uprush cannot exceed 3°C even at violent and complicated disturbance.
(4) The radio frequency generator for ablation of the present invention further includes a protection mechanism for preventing repeating ablation. The temperature of the site to be ablated is detected before each ablation is applied. If the temperature of the site to be ablated is higher than 40°C to 60°C, ablation is not started. The repeated ablation of the same site due to carelessness or misoperation of an operator can be simply and effectively avoided.

The present invention provides a device with a function of transmitting energy in the trachea and bronchus. The device can be used for delivering a direct current, an alternating current, and a radio frequency energy to a lesion, so as to remove pathologically hyperplastic bronchial smooth muscles, increase the diameter of the trachea during resting, reduce the pathological retraction and respiratory resistance of the bronchial wall, and increase the adjusting compliance of the trachea. The device can be used for the non-medicine treatment of obstructive pulmonary diseases, and for example, used for the treatment of the patients with persistent asthma, pulmonary emphysema, chronic obstructive pulmonary diseases and the like still incapable of being effectively controlled after the administration of medicine (such as corticosteroids and long-acting β receptor agonists).

### BRIEF DESCRIPTION OF THE DRAWINGS

FIG. 1 is an overall schematic diagram of a multi-electrode ablation device in Embodiment 1.
FIG. 2 shows a schematic diagram of a basket-shaped electrode assembly in Embodiment 1 in the unexpanded state.
FIG. 3 shows a schematic diagram 1 of the basket-shaped electrode assembly in Embodiment 1 in the expanded state.
FIG. 4 shows a schematic diagram 2 of the basket-shaped electrode assembly in Embodiment 1 in the expanded state.
FIG. 5 shows a local sectional view of a support component in Embodiment 1.
FIG. 6 shows a schematic diagram 1 of a balloon electrode assemblshowsy in Embodiment 2.
FIG. 7 shows a schematic diagram 2 of a balloon electrode assemblshowsy in Embodiment 2.
FIG. 8 shows a local sectional view of a support component in Embodiment 2.
FIG. 9 shows a schematic diagram of a spiral electrode assembly in Embodiment 3.
FIG. 10 shows a schematic diagram of a handle.
FIG. 11 shows an arrangement sectional view of a pressure sensor.
FIG. 12 shows a touch display screen of a radio frequency generator for ablation.
FIG. 13 shows impedance detection values of a left lobe of a first swine lung at different handle grip strengths.
FIG. 14 shows impedance detection values of a right lobe of the first swine lung at different handle grip strengths.
FIG. 15 shows impedance detection values of a left lobe of a second swine lung at different handle grip strengths.
FIG. 16 shows impedance detection values of a right lobe of the second swine lung at different handle grip strengths.
FIG. 17 shows a relationship between an electrode attachment quantity and an impedance of an isolated swine lung.
FIG. 18 shows a relationship between a saline water electrode attachment quantity and an impedance.
FIG. 19 shows detection values of radio frequency ablation on an impedance.
FIG. 20 shows a tissue impedance change curve of an animal test ablation process.
FIG. 21 shows tissue temperature and radio frequency output power curves of an ablation process without adopting segmentation control and temperature dynamic smoothening.
FIG. 22 shows tissue temperature and radio frequency output power curves of an ablation process after adoption of segmentation control and temperature dynamic smoothening.
FIG. 23 shows a record of ablation stop in an ablation process in an animal test when a tissue temperature is higher than an over temperature threshold value.

### DETAILED DESCRIPTION

A radio frequency generator for ablation of the present invention is able to generate and control a direct current, an alternating current and a radio frequency energy, collect, process and display a temperature, impedance or tension signal, and determine the ablation effectiveness according to the change of the impedance or tension signal, and the change of the impedance is one or more of a falling value of impedance, a change rate of impedance, the change in the change rate of impedance, or the change of impedance from falling to rising. Further, the ablation is determined to be effective when the falling value of impedance exceeds 10 Ω to 100 Ω, or the change rate of impedance is higher than -1 Ω/s to -50 Ω/s, or the change of impedance is from falling to rising.

The radio frequency generator for ablation of the present invention uses a segmentation control method to adjust a radio frequency output power through a closed-loop control system so as to control an ablation temperature, and the segmentation control includes: (1) a fast heating stage: lasting for 0.5 s to 2 s from the beginning of ablation, where an end point temperature of the fast heating stage reaches 50% to 80% of the ablation temperature; (2) a slow heating stage: lasting for 0.5 s to 2 s after the fast heating stage, where an end point temperature of the slow heating stage reaches 70% to 99% of the ablation temperature, or is 0.1°C to 10°C lower than the ablation temperature; and (3) a stability maintenance stage: stably maintaining the temperature after the slow heating stage until the ablation stops.

At the same time, the radio frequency generator for ablation performs dynamic smoothing on the temperature in the process of controlling the ablation temperature, including averaging, weighted averaging or median averaging on a sampling temperature value, the radio frequency generator for ablation is guided to adjust the radio frequency power output according to a temperature value obtained through dynamic smoothing, and the smooth change of the radio frequency output power in the ablation process is thus ensured. An upper limit of a threshold value of the dynamic smoothing is 0.1°C/s to 20°C/s, and a lower limit of the threshold value is -0.1°C/s to -20°C/; when the temperature change rate is smaller than the lower limit of the threshold value, a smoothing time window is prolonged; when the temperature change rate is greater than the upper limit of the threshold value, the smoothing time window is shortened; and when the temperature change rate is between the lower limit and the upper limit of the threshold value, the smoothing time window remains unchanged. The upper limit of the threshold value of the dynamic smoothing is 5°C/s, and the lower limit is -5°C/s. A dynamic range of the smoothing time window is 0 s to 10 s, preferably 0 s to 2.5 s.

Further, the radio frequency generator for ablation further includes a protection mechanism for preventing repeating ablation. A temperature of a site to be ablated is detected before each ablation is applied. If the temperature of the site to be ablated is higher than 40°C to 60°C, ablation is not started.

Further, the radio frequency generator for ablation includes a radio frequency energy transmission/feedback control mechanism: after the radio frequency energy output for 2 to 4 s, a temperature of an ablated tissue reaches a set temperature and is maintained for 6 to 8 s, an over temperature alarm is given when the temperature of the ablated tissue is higher than an over temperature threshold value, and an ablation system automatically stops the radio frequency energy output. The set temperature ranges from 60°C to 70°C, and the over temperature threshold value is 1°C to 10°C higher than the set temperature. Preferably, the set temperature is 65°C, and the over temperature threshold value is 3°C higher than the set temperature.

The objective of the present invention can be achieved by using the multi-electrode ablation device of the embodiment of the present invention. The following embodiments are merely exemplary embodiments of the present invention and are not intended to limit the present invention in any way. Any simple modifications, equivalent variations and modifications made on the above embodiments, within the scope of appended claims, are still within the scope of the techniques and methods of the solution of the present invention.

### Embodiment 1

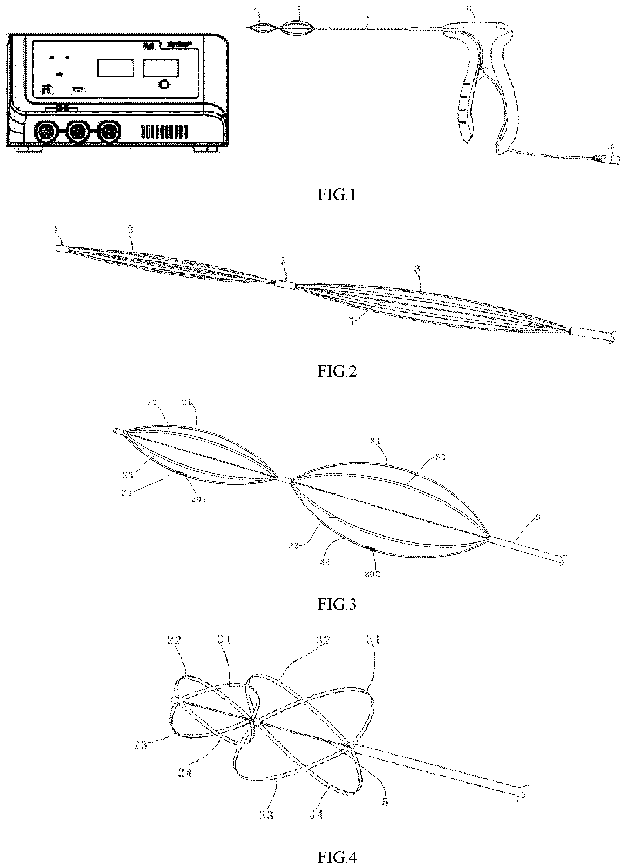

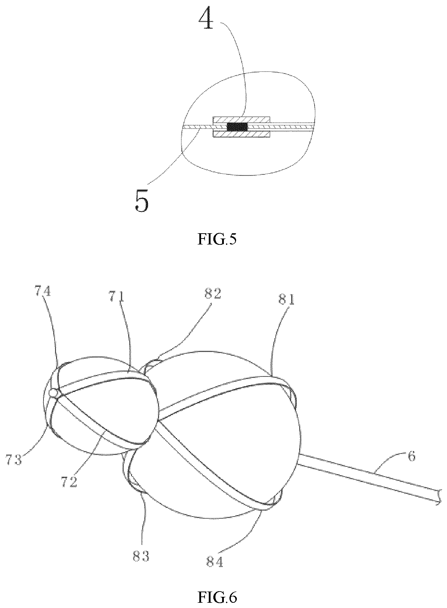

The present invention relates to a device for achieving a function of transmitting energy in the trachea and bronchus, and further relates to a multi-electrode ablation device. As shown in FIG. 1, the device mainly includes a first electrode assembly 2, a second electrode assembly 3, a guiding catheter body 6, a handle 17 and a connector 18. As shown in FIG. 2, the first electrode assembly 2 and the second electrode assembly 3 are continuously disposed in an axial direction of the guiding catheter body 6, and a damage-prevention structure 1 is disposed at a head end of the electrode assembly and is configured to fix the first electrode assembly 2 at the same time. The first electrode assembly 2 and the second electrode assembly 3 are connected to each other through a support component 4, a near end of the first electrode assembly 2 and a far end of the second electrode assembly 3 are fixed to the support component 4, a far end of a traction steel wire 5 is connected to the damage-prevention structure 1 at the head end, and a near end is fixed to the support component 4 (as shown in FIG. 5), and enters the handle 17 through the guiding catheter body 6. A near end of the second electrode assembly 3 is fixed to the catheter body 6. As shown in FIG. 3, when the handle 17 controls the traction steel wire 5 to contract towards the near end, the first electrode assembly 2 is driven to expand first, at the same time, the second electrode assembly 3 synchronously expands, according to the characteristics of the trachea tract, the electrode assembly is designed to be smaller at the far end, and larger at the near end, and a diameter difference is about 1 to 5 mm.

The first electrode assembly 2 and the second electrode assembly 3 are provided with a plurality of electrodes: a first electrode 21, a second electrode 22, a third electrode 23, a fourth electrode 24, a fifth electrode 31, a sixth electrode 32, a seventh electrode 33 and an eighth electrode 34, the electrodes are made of stainless steel materials, and have certain elasticity, each electrode is connected to the handle through an independent electrode conductor, and the handle is transmitted to a bronchial radio frequency generator for ablation through the connector 18. In use, each electrode forms a loop with a control circuit board through a trachea tissue, and each electrode can independently detect an attachment impedance value of the electrode and the tissue. When the electrode is well attached (the detected impedance value is 500 Ω to 1000 Ω), the bronchial radio frequency generator for ablation will send radio frequency energy to ablate the lesion tissue, a temperature sensor 201 and a temperature sensor 202 are respectively disposed on the first electrode assembly 2 and the second electrode assembly 3, and can independently detect the temperature of the tissue around the corresponding electrode assembly.

### Embodiment 2

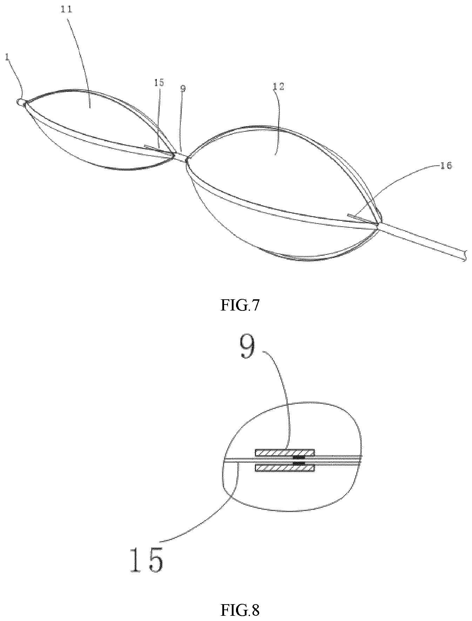

Devices as shown in FIG. 6 to FIG. 8 are a second embodiment of the present invention, a first balloon 11 and a second balloon 12 are disposed under the first electrode assembly 2 and the second electrode assembly 3, a near end of the first balloon 11 is provided with a balloon first air passage 15, and a near end of the second balloon 12 is provided with a balloon second air passage 16. The first balloon 11 and the second balloon 12 are isolated from each other, and the first air passage 15 and the second air passage 16 independently provide air for the first balloon 11 and the second balloon 12. When the air enters the balloons through the balloon air passages, a first electrode 71, a second electrode 72, a third electrode 73, a fourth electrode 74, a fifth electrode 81, a sixth electrode 82, a seventh electrode 83 and an eighth electrode 84 expand under pressure, the electrode assemblies expand, the air inflow is controlled by an external air inlet apparatus, the expansion size of the electrode assemblies can be set through the air inflow, and the first electrode assembly 2 and the second electrode assembly 3 are independently controlled to adapt to the requirements of different sizes of the trachea lesion sites.

The first electrode 71, the second electrode 72, the third electrode 73, the fourth electrode 74, the fifth electrode 81, the sixth electrode 82, the seventh electrode 83 and the eighth electrode 84 are provided with independent electrode conductors. In use, each electrode forms a loop with a control circuit board through the trachea tissue, and each electrode can independently detect an attachment impedance value of the electrode and the tissue. A temperature sensor 201 and a temperature sensor 202 are respectively disposed on the electrode assembly 2 and the electrode assembly 3, and can independently detect the temperature of the tissue around the corresponding electrode assembly.

### Embodiment 3

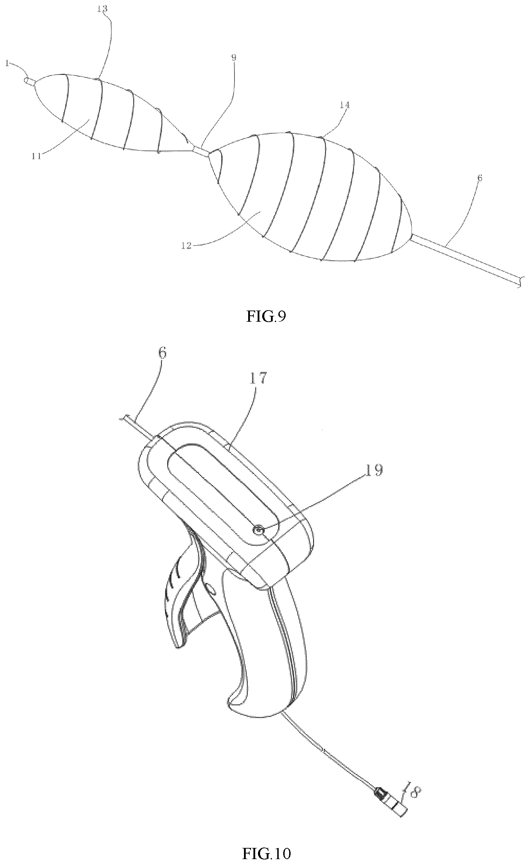

The device as shown in FIG. 9 is a third embodiment, a first annular electrode 1 and a second annular electrode 2 are spirally disposed on a first balloon 11 and a second balloon 12. When the balloons are inflated, outer diameters of the first annular electrode 1 and the second annular electrode 2 are increased. Independent electrode conductors are disposed on the first annular electrode 1 and the second annular electrode 2. In use, each electrode forms a loop with a control circuit board through a trachea tissue, and each electrode can independently detect an attachment impedance value of the electrode and the tissue. A temperature sensor 201 and a temperature sensor 202 are respectively disposed on the annular electrode 1 and the annular electrode 2, and can independently detect the temperature of the tissue around the corresponding electrode assembly.

As shown in FIG. 10, an indicating lamp 19 is disposed on a handle 17. Theoretically, an impedance value of 500 Ω to 1000 Ω or below after the electrode is attached to the tissue indicates that the radio frequency ablation can be performed. When a bronchial radio frequency generator for ablation detects that the electrode attachment impedance value is 500 Ω to 1000 Ω or below, the indicating lamp becomes green, indicating that the ablation can be performed. When the bronchial radio frequency generator for ablation detects that the electrode attachment impedance value is 500 Ω to 1000 Ω or above, the indicating lamp is red, indicating that the discharging ablation cannot be performed.

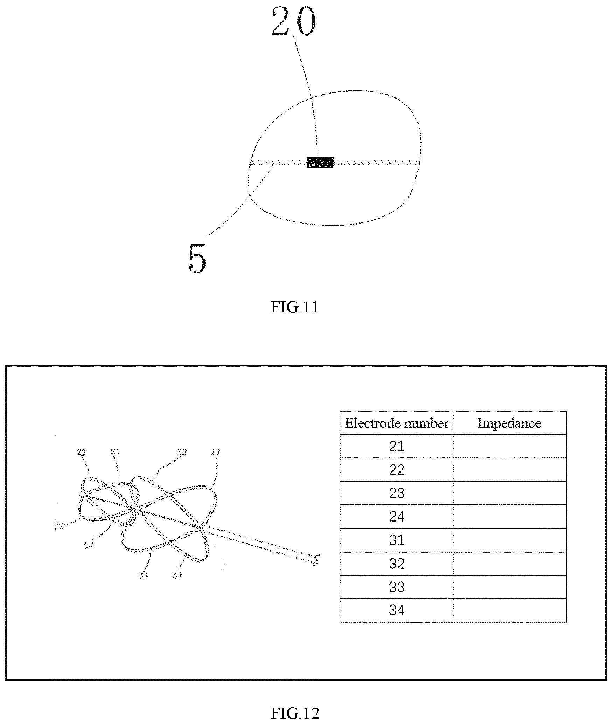

As shown in FIG. 11, a pressure sensor 20 is disposed in a local area of a traction steel wire 5, two ends of the pressure sensor are respectively connected to two ends of the traction steel wire, when the electrode assembly is dragged, the traction steel wire 5 is stressed, at this moment, the pressure sensor 20 will receive the same tension, through the treatment by the bronchial radio frequency generator for ablation, the tension will be displayed to determine the attachment degree. When the electrode attaches to the tissue, the attachment degree of an electrode arm to the tissue can be determined through determining the traction tension.

As shown in FIG. 12, the radio frequency generator for ablation is provided with a touch display screen for displaying a state of electrodes and an adhesion impedance value of the electrodes and the tissue, and one or a plurality of electrodes are able to be controlled to release the energy by clicking the touch display screen.

The guiding catheter body 6 can be served as a guiding tube, the guiding tube is provided with a tube cavity accommodating the electrode assembly 2 and the electrode assembly 3, the electrode assembles can freely extend and retract in the guiding tube, and liquid, such as anti-inflammatory medicine and anaesthetics can enter the ablation lesion tissue through the tube cavity of the guiding tube so as to relive the pain and complications of a patient.

### Embodiment 4 Investigation on relationship among impedance of multi-electrode ablation device of the present invention, electrode quantity and tension

Clinic application of the multi-electrode ablation device was simulated through isolated tissue tests, and the impedance detection values of an ablation catheter under the conditions of different bronchus sites, different handle grip strengths and different electrode attachment quantities were observed.

Test environment: temperature: 15°C to 20°C; and humidity: 55% RH to 60% RH.

Test tissue: 2 fresh isolated swine lungs.

Test principle: the isolated swine lungs were soaked in saline water, the ablation catheter was connected onto the radio frequency generator for ablation, the ablation catheter was operated, and the impedance display values on the radio frequency generator for ablation were observed and recorded under the conditions of different bronchus sites, different handle grip strengths and different electrode attachment quantities.

Test sites: superior lobe of left lung, inferior lobe of left lung, superior lobe of right lung, and inferior lobe of right lung.

### 1. Investigation on relationship between different electrode tension and impedance

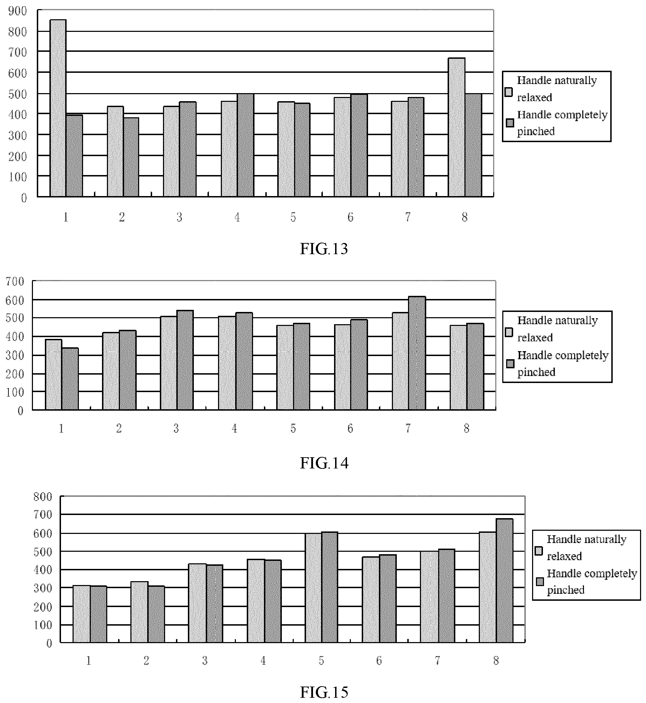

The impedance detection values under the conditions of the naturally relaxed state and the completely pinched state of the handle of the catheter at different bronchus sites are observed and recorded the results are as shown in Tables 1 to 4 and FIGs 13 to 16: The results show that the electrode tension is associated with the impedance detection values.

**Table 1 Impedance detection values of left lobe of the first swine lung at different handle grip strengths**

| Serial number | Test site | Impedance (Ω) | | Impedance change (Ω) |
|---|---|---|---|---|
| | | Handle naturally relaxed | Handle completely pinched | |
| 1 | Superior lobe of left lung | 850 | 390 | 460 |
| 2 | Superior lobe of left lung | 433 | 383 | 50 |
| 3 | Superior lobe of left lung | 433 | 456 | -23 |
| 4 | Superior lobe of left lung | 463 | 500 | -37 |
| 5 | Inferior lobe of left lung | 453 | 448 | 5 |
| 6 | Inferior lobe of left lung | 478 | 494 | -16 |
| 7 | Inferior lobe of left lung | 461 | 478 | -17 |
| 8 | Inferior lobe of left lung | 671 | 496 | 175 |

**Table 2 Impedance detection values of right lobe of the first swine lung at different handle grip strengths**

| Serial number | Test site | Impedance (Ω) | | Impedance change (Ω) |
|---|---|---|---|---|
| | | Handle naturally relaxed | Handle completely pinched | |
| 1 | Superior lobe of right lung | 380 | 340 | 40 |
| 2 | Superior lobe of right lung | 418 | 430 | -12 |
| 3 | Superior lobe of right lung | 507 | 540 | -33 |
| 4 | Superior lobe of right lung | 512 | 530 | -18 |
| 5 | Inferior lobe of right lung | 460 | 470 | -10 |
| 6 | Inferior lobe of right lung | 467 | 490 | -23 |
| 7 | Inferior lobe of right lung | 530 | 620 | -90 |
| 8 | Inferior lobe of right lung | 460 | 470 | -10 |

**Table 3 Impedance detection values of left lobe of the second swine lung at different handle grip strengths**

| Serial number | Test site | Impedance (Ω) | | Impedance change (Ω) |
|---|---|---|---|---|
| | | Handle naturally relaxed | Handle completely pinched | |
| 1 | Superior lobe of left lung | 315 | 305 | 10 |
| 2 | Superior lobe of left lung | 334 | 305 | 29 |
| 3 | Superior lobe of left lung | 428 | 425 | 3 |
| 4 | Superior lobe of left lung | 458 | 450 | 8 |
| 5 | Inferior lobe of left lung | 596 | 601 | -5 |
| 6 | Inferior lobe of left lung | 467 | 480 | -13 |
| 7 | Inferior lobe of left lung | 496 | 510 | -14 |
| 8 | Inferior lobe of left lung | 604 | 678 | -74 |

**Table 4 Impedance detection values of right lobe of the second swine lung at different handle grip strengths**

| Serial number | Test site | Impedance (Ω) | | Impedance change (Ω) |
|---|---|---|---|---|
| | | Handle naturally relaxed | Handle completely pinched | |
| 1 | Superior lobe of right lung | 330 | 298 | 32 |
| 2 | Superior lobe of right lung | 326 | 315 | 11 |
| 3 | Superior lobe of right lung | 350 | 320 | 30 |
| 4 | Superior lobe of right lung | 355 | 320 | 35 |
| 5 | Inferior lobe of right lung | 300 | 308 | -8 |
| 6 | Inferior lobe of right lung | 320 | 329 | -9 |
| 7 | Inferior lobe of right lung | 370 | 370 | 0 |
| 8 | Inferior lobe of right lung | 384 | 400 | -16 |
| 9 | Inferior lobe of right lung | 410 | 428 | -18 |
| 10 | Inferior lobe of right lung | 380 | 365 | 15 |

### 2. Investigation on relationship between different electrode attachment quantities and impedance

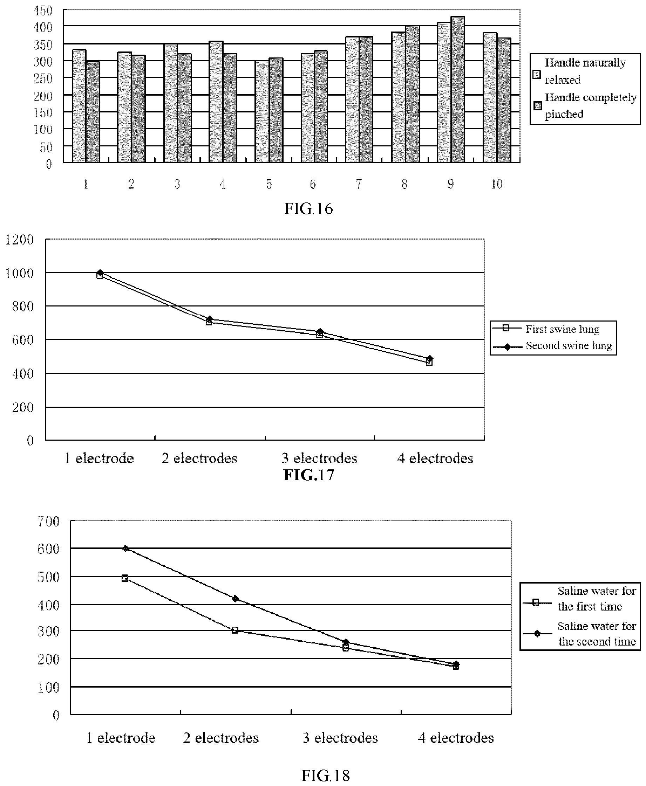

Different quantities of electrodes are attached to the bronchus, the impedance detection values are observed and recorded, and the results are as shown in Table 5 and FIG. 17. Different quantities of electrodes are soaked into saline water, the impedance detection values are observed and recorded (with the influence of the attachment pressure excluded), and the results are as shown in Table 6 and FIG. 18. The results show that different electrode attachment quantities have obvious influence on the impedance detection values, the more the number of electrodes attached, the smaller the impedance detection value, and the electrode attachment quantity can be determined according to the impedance detection value.

**Table 5 Relationship between electrode attachment quantity and impedance of isolated swine lung**

| Serial number | Impedance (Ω) | | | |
|---|---|---|---|---|
| | 1 electrode | 2 electrodes | 3 electrodes | 4 electrodes |
| The first swine lung | 980 | 700 | 630 | 460 |
| The second swine lung | 999 | 720 | 650 | 490 |

**Table 6 Relationship between electrode attachment quantity and impedance of saline water**

| Serial number | Impedance (Ω) | | | |
|---|---|---|---|---|
| | 1 electrode | 2 electrodes | 3 electrodes | 4 electrodes |
| Saline water for the first time | 490 | 300 | 240 | 170 |
| Saline water for the second time | 600 | 420 | 260 | 180 |

### 3. Investigation on influence of radio frequency ablation on impedance

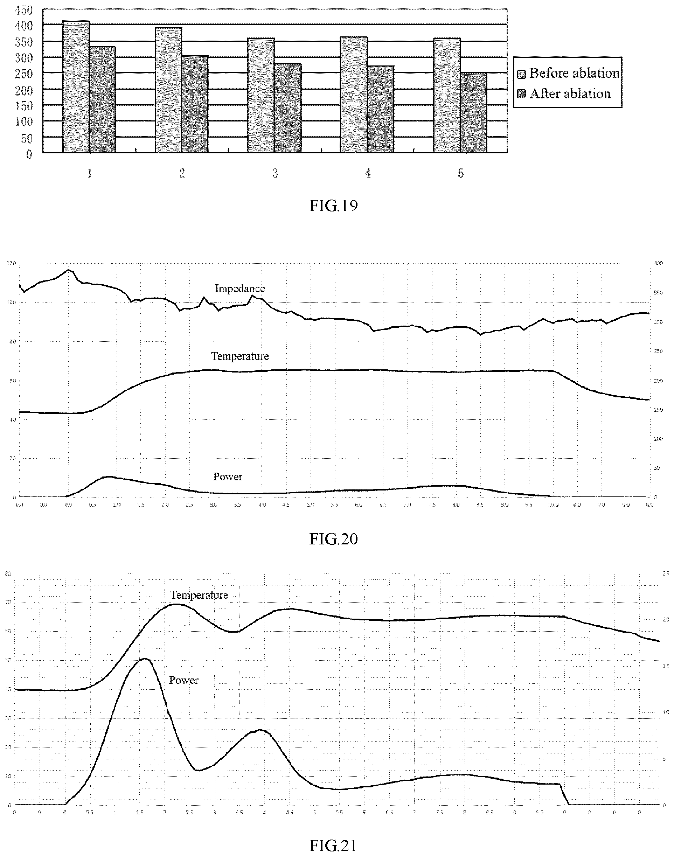

The radio frequency is output, the impedance detection values are observed and recorded, the results are as shown in Table 7 and FIG. 19, and the results show that the radio frequency ablation causes impedance detection value falling, the ablation effectiveness can be determined according to the change of an impedance or tension signal, and the change of the impedance is one or more of the falling value of impedance, the change rate of impedance, the change in the change rate of impedance, or the change of impedance from falling to rising.

**Table 7 Detection values of radio frequency ablation on impedance**

| Serial number | Test conditions | Impedance (Ω) | | Impedance change (Ω) | Highest temperature (°C) |
|---|---|---|---|---|---|
| | | Before ablation | After ablation | | |
| 1 | 18 W, 10 s | 411 | 330 | 81 | 50 |
| 2 | 18 W, 10 s | 392 | 302 | 90 | 60 |
| 3 | 18 W, 10 s | 360 | 279 | 81 | 67 |
| 4 | 18 W, 15 s | 363 | 272 | 91 | 80 |
| 5 | 65°C, 15s | 360 | 252 | 108 | 66 |

### Embodiment 5 Investigation on ablation effectiveness of multi-electrode ablation device of present invention

The ablation effectiveness of the multi-electrode ablation device of the present invention is investigated by using an animal test. A logic relationship among the impedance, power and temperature is defined, the generated and controlled direct current, alternating current and radio frequency energy are precisely controlled, a temperature, impedance or tension signal is collected, processed and displayed, and the ablation effectiveness is determined according to the change of the impedance signal. The ablation was determined to be effective when a falling value of impedance exceeded 10 Ω to 100 Ω, or a change rate of impedance is higher than -1 Ω/s to -50 Ω/s, or the change of impedance is from falling to rising.

Specific operations are as follows:
Electrodes of the multi-electrode ablation device of the present invention was put into a site to be tested of a dog lung, and a data interface of the multi-electrode ablation device was connected to a computer. The multi-electrode ablation device was operated for ablation. The computer displayed and recorded the temperature, power and impedance data in the test process. A whole process of the test process was observed by using a bronchial endoscope.

The results are as shown in FIG. 20. FIG. 20 is a tissue impedance change curve of an ablation process in animal tests. The abscissa is the time, the left ordinate is the tissue temperature and the radio frequency output power, and the right ordinate is the tissue impedance. As shown in the figure, after the ablation is started, the tissue impedance starts to fall, additionally, the tissue impedance falling speed was gradually decelerated, and then the tissue impedance gradually starts to rise, indicating that the ablation of the multi-electrode ablation device of the present invention is effective.

### Embodiment 6 Investigation on safety and temperature anti-interference capability of multi-electrode ablation device of present invention

The present invention relates to a device with a function of transmitting energy in the trachea and bronchus, and the device uses a segmentation proportional integral control algorithm to perform dynamic smoothening on the temperature. 0 s to 1 s from the beginning of the ablation is a fast heating stage, the radio frequency output power rises fast to be 10 W or above from 0, and the tissue temperature starts to rise fast. 1 s to 2 s is a slow heating stage, the radio frequency output power slowly rises, and starts to gradually fall, and the tissue temperature heating speed starts to be decelerated. After such 2 s till the ablation stop is a stable maintenance stage, and the radio frequency output power slowly falls and is adjusted slightly so as to maintain the tissue temperature.

A dynamic range of a temperature dynamic smoothening time window is 0 s to 2.5 s. Each time when a temperature change rate is greater than 5°C/s, the smoothing time window is shortened by 0.01 s. Each time when the temperature change rate is smaller than -5°C/s, the smoothening time window is prolonged by 0.01 s. The temperature change rate is between -5°C/s and 5°C/s, and the smoothening time window remains unchanged. The temperature in the smoothening time window is subjected to average calculation to thus achieve the temperature dynamic smoothening.

The operations of the animal test are the same as those in Embodiment 5.

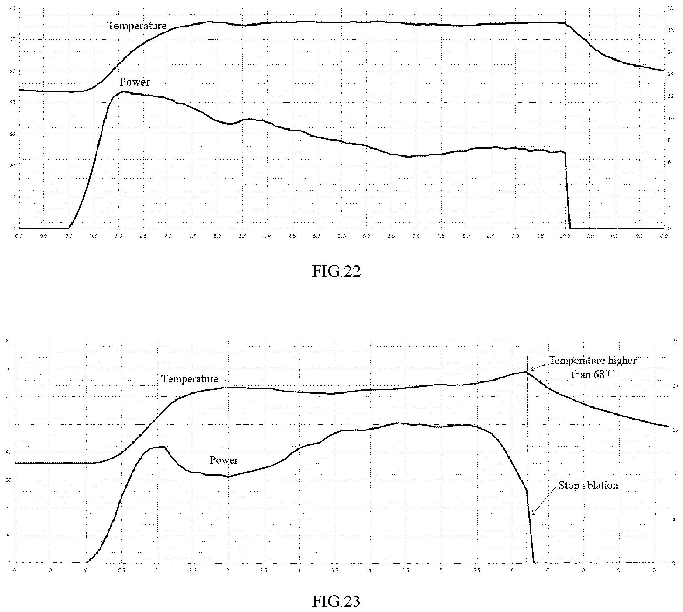

As shown in FIG. 21 and FIG. 22, FIG. 21 shows tissue temperature and radio frequency output power curves of an ablation process without adopting segmentation control and temperature dynamic smoothening in the animal test. FIG. 22 shows tissue temperature and radio frequency output power curves of an ablation process after adoption of segmentation control and temperature dynamic smoothening. The abscissa is the time, the left ordinate is the tissue temperature, and the right ordinate is the radio frequency output power. As shown in the figure, after the ablation is started, the radio frequency output power rises fast within 1 s, slowly rise and starts to fall within 2 s, and slowly fall and is adjusted slight after 2 s. After the ablation is started, the tissue temperature starts to rise fast within 1 s, slowly rise within 2 s, and reaches the ablation temperature within 3 s and maintains at the ablation temperature. The device controlled the radio frequency output power so that the temperature of the ablation electrodes reached the ablation temperature within 3 s. Additionally, after the ablation temperature is reached, the temperature uprush is less than 1°C, the tissue temperature is stably maintained at the ablation temperature, and the fluctuation is smaller than 1°C. In the whole ablation treatment process, the radio frequency output power smoothly changes without suddenly applied and (or) suddenly changed radio frequency energy. When the segmentation control and temperature dynamic smoothening are not adopted, the tissue temperature generates obvious oscillation, and the temperature uprush is greater. After the segmentation control and temperature dynamic smoothening are adopted, the tissue temperature is kept stable, and the temperature uprush is smaller.

The results shows that the radio frequency output power is successfully adjusted by using the closed loop control system by the segmentation control method to control the ablation temperature, and the temperature dynamic smoothening is utilized to overcome various kinds of disturbances. Therefore, the safety and the effectiveness of the system are further ensured, i.e., the conditions of wrong ablation or ablation incapability cannot occur, and the condition of repeated ablation or excessive ablation cannot occur.

### Embodiment 7 Investigation on safety control capability of radio frequency ablation device of present invention

The radio frequency ablation device of the present invention includes a radio frequency energy transmission/feedback control mechanism: after the radio frequency energy output for 2 to 4 s, a temperature of an ablated tissue reached a set temperature of 60°C to 70°C and is maintained for 6 to 8 s, an over temperature alarm is given when the temperature of the ablated tissue is higher than an over temperature threshold value (1°C to 10°C higher than the set temperature), and an ablation system automatically stops the radio frequency energy output.

The operations of the animal test are the same as those in Embodiment 5.

The results are as shown in FIG. 23. FIG. 23 shows a record of ablation stop in an ablation process in an animal test when a tissue temperature is higher than an over temperature threshold value. The abscissa is the time, the left ordinate is the tissue temperature, and the right ordinate is the radio frequency output power. As shown in the figure, the tissue temperature is higher than 68°C, the radio frequency output power falls fast to 0, and the ablation is stopped.

## Claims

1. A radio frequency generator for ablation for use in a device for transmitting energy in the trachea and bronchus, the generator being able to
generate and control a direct current, an alternating current and a radio frequency energy,
collect, process and display a temperature, impedance or tension signal, and
determine the ablation effectiveness according to the change of the impedance or tension signal, and the change of the impedance is one or more of
a falling value of impedance,
a change rate of impedance,
the change in the change rate of impedance, or
the change of impedance from falling to rising,
wherein the radio frequency generator for ablation is configured for using a segmentation control method to adjust a radio frequency output power through a closed-loop control system so as to control an ablation temperature, and the segmentation control comprises:
(1) a fast heating stage: lasting for 0.5 s to 2 s from the beginning of ablation, wherein an end point temperature of the fast heating stage reaches 50% to 80% of the ablation temperature;
(2) a slow heating stage: lasting for 0.5 s to 2 s after the fast heating stage, wherein an end point temperature of the slow heating stage reaches 70% to 99% of the ablation temperature, or is 0.1°C to 10°C lower than the ablation temperature; and
(3) a stability maintenance stage: stably maintaining the temperature after the slow heating stage until the ablation stops.

2. The radio frequency generator for ablation according to claim 1, wherein the ablation is determined to be effective when a falling value of impedance exceeds 10 Ω to 100 Ω, or the change rate of impedance is higher than -1 Ω/s to -50 Ω/s, or the change of impedance is from falling to raising;
optionally wherein the ablation is determined to be effective when the falling value of impedance exceeds 20 Ω to 50 Ω, or the change rate of impedance is higher than -5 Ω/s to - 50 Ω/s, or the change of impedance is from falling to raising.

3. The radio frequency generator for ablation according to claim 1, wherein the segmentation control comprises: (1) the fast heating stage: lasting for 1 s from the beginning of ablation, wherein an end point temperature of the fast heating stage reaches 65% of the ablation temperature; (2) the slow heating stage: lasting for 1 s after the fast heating stage, wherein an end point temperature of the slow heating stage reaches 90% of the ablation temperature, or is 2°C lower than the ablation temperature; and (3) the stability maintenance stage: stably maintaining the temperature after the slow heating stage until the ablation stops.

4. The radio frequency generator for ablation according to claim 1, wherein the radio frequency generator for ablation is configured to perform dynamic smoothing on the temperature in the process of controlling the ablation temperature, comprising averaging, weighted averaging or median averaging on a sampling temperature value, the radio frequency generator for ablation being guided to adjust the radio frequency power output according to a temperature value obtained through dynamic smoothing, and the smooth change of the radio frequency output power in the ablation process is thus ensured.

5. The radio frequency generator for ablation according to claim 4, wherein an upper limit of a threshold value of the dynamic smoothing is 0.1°C/s to 20°C/s, and a lower limit of the threshold value is -0.1 °C/s to -20°C/; when a temperature change rate is smaller than the lower limit of the threshold value, a smoothing time window is prolonged; when the temperature change rate is greater than the upper limit of the threshold value, the smoothing time window is shortened; and when the temperature change rate is between the lower limit and the upper limit of the threshold value, the smoothing time window remains unchanged.

6. The radio frequency generator for ablation according to claim 5, wherein the upper limit of the threshold value of the dynamic smoothing is 5°C/s, and the lower limit is -5°C/s;
or wherein a dynamic range of the smoothing time window is from 0 s to 10 s, optionally wherein a dynamic range of the smoothing time window is from 0 s to 2.5 s.

7. The radio frequency generator for ablation according to any one of claims 1 to 6, further comprising a protection mechanism for preventing repeating ablation, wherein a temperature of a site to be ablated is detected before each ablation is applied, and if the temperature of the site to be ablated is higher than 40°C to 60°C, ablation is not started; optionally wherein the temperature of the site to be ablated is detected before each ablation is applied, and if the temperature of the site to be ablated is higher than 45°C, ablation is not started.

8. The radio frequency generator for ablation according to claim 1, configured for using
a method 1 comprising: detecting the impedance by a continuous weak alternating current signal, and calculating the impedance through a voltage and a current during radio frequency output; and/or
a method 2 comprising: directly detecting the impedance without radio frequency output.

9. The radio frequency generator for ablation according to claim 1, comprising a radio frequency energy transmission/feedback control mechanism configured for: after the radio frequency energy output for 2 to 4 s, a temperature of an ablated tissue reaches a set temperature and is maintained for 6 to 8 s, an over temperature alarm is given when the temperature of the ablated tissue is higher than an over temperature threshold value, and an ablation system automatically stops the radio frequency energy output;
optionally wherein the set temperature ranges from 60°C to 70°C, and the over temperature threshold value is 1°C to 10°C higher than the set temperature;
more optionally wherein the set temperature is 65°C, and the over temperature threshold value is 3°C higher than the set temperature.

10. The radio frequency generator for ablation according to claim 1, adopting a design of multiple central controllers, dual temperature circuits, and dual voltage and current circuits; or
wherein the radio frequency generator for ablation is provided with a data transmission interface, and is able to be externally connected to a computer to obtain information of various parameters in real time; or
wherein the radio frequency generator for ablation is provided with a touch display screen for displaying a state of electrodes and an adhesion impedance value of the electrodes and a tissue, and one or a plurality of electrodes are able to be controlled to release the energy by clicking the touch display screen.

11. A multi-electrode ablation device, comprising the radio frequency generator for ablation according to any one of claims 1 to 10, an electrode assembly (2, 3), a guiding catheter (6), a handle (17) and a connector (18),
wherein at least one cavity is formed in the guiding catheter (6);
wherein the electrode assembly (2, 3) is disposed at a front end of the guiding catheter (6), and is connected to the handle (17) by passing a circuit through the inside of the guiding catheter (6), the electrode assembly (2, 3) including one or more electrode groups and one or more detection devices, the electrode group being able to apply an electric energy, a radio frequency energy, a laser energy, high-density focused ultrasound or a low temperature for ablation, and the detection device being configured to detect a temperature, impedance or tension;
wherein the handle (17) is connected to the connector (18) and one or more group of electrode assemblies (2, 3), and comprises one or a plurality of operation components configured to control constriction, expansion and energy release of the electrode groups, and is able to control the electrode assembly (2, 3) to extend out of or retreat back into the guiding catheter (6); and
wherein the connector (18) is configured to provide an energy for electrodes.

12. The multi-electrode ablation device according to claim 11, wherein the detection device comprises a temperature detection device, an impedance detection device and a tension detection device.

13. The multi-electrode ablation device according to claim 12, wherein the electrode group (2, 3) comprises one or a plurality of electrodes, each electrode being connected to the handle (17) through an independent electrode conductor,
wherein the electrode group is configured to expand in a basket shape, spiral shape or balloon shape under the control of the operation component, and in presence of a plurality of electrode groups, the electrode groups are sequentially arranged in series;
and wherein the closer the electrode groups are to the handle (17), the larger an outer diameter after expansion, and the outer diameter is 1 to 20 mm.

14. The multi-electrode ablation device according to claim 13, wherein the electrode assembly (2, 3) further comprises a traction steel wire (5), two ends of the electrode being fixed to the traction steel wire (5), the traction steel wire (5) passing through the guiding catheter (6) to be connected to the handle (17), and the handle controlling contraction and expansion of the electrode group by pulling and releasing the traction steel wire (5); optionally wherein in presence of a plurality of electrode groups, a damage-prevention structure (1) is disposed at a head end of the electrode group at an end farthest from the handle (17), and the electrode groups are connected through support components; optionally wherein a pressure sensor (20) is disposed on the traction steel wire (5);
or wherein the electrode assembly (2, 3) further comprises a balloon (11) disposed between the electrodes, wherein the balloon is configured to pass through the guiding catheter (6) through a balloon air passage to be connected to the handle (17), and is able to be connected to an air inlet apparatus through the handle (17), and the electrode group is configured to expand after the balloon (11) is inflated to expand; and in presence of a plurality of electrode groups, a plurality of balloons (11, 12) are sequentially arranged in series, and the plurality of balloons are respectively connected to the handle (11, 12) through independent balloon air passages (15, 16).

15. The multi-electrode ablation device according to claim 11, wherein the closer the guiding catheter (6) is to the handle (17), the greater the hardness is, and the hardness distribution ranges from 90 A to 80 D of a Shore hardness; or
wherein the operation component of the handle (17) comprises a control circuit board and a control button; the control circuit being connected to the electrode assembly (2, 3) and the control button, and the control button being configured to control different components in different electrode assemblies respectively, optionally wherein the electrode group is able to control one or a plurality of electrodes to release an energy under the control of the operation component of the handle; or
wherein the radio frequency generator for ablation is able to display an impedance or tension of the electrode and indicate whether the electrode assembly is well attached to the tracheal wall or not: an impedance value smaller than or equal to the threshold value after electrodes of a bronchial radio frequency generator for ablation is attached to the tissue indicating good attachment of the electrode assembly to the bronchial wall, optionally wherein the threshold value of impedance ranges from 500 Ω to 1000 Ω, more optionally wherein the threshold value of impedance is 900 Ω; or
wherein according to a method of determining whether the electrodes are in well contact with the tracheal wall or not by the radio frequency generator for ablation, the radio frequency generator for ablation is able to measure the impedance of each electrode, and if the impedance is consistent, the contact between the electrodes and the tracheal wall is good; and if the contact between a certain electrode and the tracheal wall is not good, the impedance is different from that of others in good contact.

## Patentansprüche

1. Radiofrequenzgenerator für eine Ablation zur Verwendung in einer Vorrichtung zum Übertragen von Energie in die Trachea und die Bronchie, wobei der Generator dazu in der Lage ist, zum:
Erzeugen und Steuern eines Gleichstroms, eines Wechselstroms und einer Radiofrequenzenergie,
Erfassen, Verarbeiten und Anzeigen einer Temperatur, einer Impedanz oder eines Tensionssignals, und
Bestimmen der Ablationseffizienz gemäß der Änderung der Impedanz oder des Tensionssignals, und wobei die Änderung der Impedanz eines oder mehrere ist/sind aus:
einem sinkenden Wert einer Impedanz,
einer Änderungsrate einer Impedanz,
der Änderung in der Änderungsrate einer Impedanz, oder
der Änderung einer Impedanz von sinkend zu steigend,
wobei der Radiofrequenzgenerator für eine Ablation dazu eingerichtet ist, ein Segmentationssteuerungsverfahren zu verwenden, um eine Radiofrequenz-Ausgabeenergie durch ein geschlossenes Steuerungssystem anzupassen, um eine Ablationstemperatur zu steuern, und wobei die Segmentationssteuerung umfasst:
(1) eine Phase eines schnellen Erwärmens: welche für 0,5 s bis 2 s von dem Beginn einer Ablation andauert, wobei eine Endpunkttemperatur der Phase eines schnellen Erwärmens 50% bis 80% der Ablationstemperatur erreicht;
(2) eine Phase eines langsamen Erwärmens: welche für 0,5 s bis 2 s nach der Phase eines schnellen Erwärmens andauert, wobei eine Endpunkttemperatur der Phase eines langsamen Erwärmens 70% bis 99% der Ablationstemperatur erreicht oder 0,1°C bis 10°C niedriger als die Ablationstemperatur ist; und
(3) eine Phase einer Stabilitätserhaltung: welche die Temperatur nach der Phase eines langsamen Erwärmens bis die Ablation stoppt stabil hält.

2. Radiofrequenzgenerator für eine Ablation nach Anspruch 1, wobei die Ablation als effizient bestimmt wird, wenn ein sinkender Wert einer Impedanz 10 Ω bis 100 Ω übersteigt oder die Änderungsrate einer Impedanz größer als -1 Ω/s bis -50 Ω/s beträgt oder die Änderung einer Impedanz von sinkend zu steigend ist;
optional wobei die Ablation als effizient bestimmt wird, wenn der sinkende Wert einer Impedanz 20 Ω bis 50 Ω übersteigt oder die Änderungsrate einer Impedanz größer als -5 Ω/s bis -50 Ω/s beträgt oder die Änderung einer Impedanz von sinkend zu steigend ist.

3. Radiofrequenzgenerator für eine Ablation nach Anspruch 1, wobei die Segmentationssteuerung umfasst: (1) die Phase eines schnellen Erwärmens: welche für 1 s von dem Beginn einer Ablation andauert, wobei eine Endpunkttemperatur der Phase eines schnellen Erwärmens 65% der Ablationstemperatur erreicht; (2) die Phase eines langsamen Erwärmens: welche für 1 s nach der Phase eines schnellen Erwärmens andauert, wobei eine Endpunkttemperatur der Phase eines langsamen Erwärmens 90% der Ablationstemperatur erreicht oder 2°C niedriger als die Ablationstemperatur ist; und (3) die Phase einer Stabilitätserhaltung: welche die Temperatur nach der Phase eines langsamen Erwärmens bis die Ablation stoppt stabil hält.

4. Radiofrequenzgenerator für eine Ablation nach Anspruch 1, wobei der Radiofrequenzgenerator für eine Ablation dazu eingerichtet ist, während des Vorgangs eines Steuerns der Ablationstemperatur ein dynamisches Glätten an der Temperatur durchzuführen, umfassend eine Mittelung, ein gewichtetes Mitteln oder ein Median-Mitteln an einem Probentemperaturwert, wobei der Radiofrequenzgenerator für eine Ablation geführt wird, um die Radiofrequenz-Energieausgabe gemäß eines Temperaturwertes anzupassen, welcher durch dynamisches Glätten erhalten wird, und wobei die glatte Änderung der Radiofrequenz-Ausgabeenergie in dem Ablationsvorgang dadurch gewährleistet ist.

5. Radiofrequenzgenerator für eine Ablation nach Anspruch 4, wobei ein oberes Limit eines Schwellenwertes des dynamischen Glättens 0,1°C/s bis 20°C/s beträgt und ein unteres Limit des Schwellenwertes -0,1°C/s bis -20°C/s beträgt; wenn eine Temperaturänderungsrate kleiner als das untere Limit des Schwellenwertes ist, wird ein Glättungszeitfenster verlängert; wenn die Temperaturänderungsrate größer als das obere Limit des Schwellenwertes ist, wird das Glättungszeitfenster verkürzt; und wenn die Temperaturänderungsrate zwischen dem unteren Limit und dem oberen Limit des Schwellenwertes ist, verbleibt das Glättungszeitfenster unverändert.

6. Radiofrequenzgenerator für eine Ablation nach Anspruch 5, wobei das obere Limit des Schwellenwertes des dynamischen Glättens 5°C/s beträgt und das untere Limit -5°C/s beträgt;
oder wobei ein dynamischer Bereich des Glättungszeitfensters von 0 s bis 10 s beträgt, optional wobei ein dynamischer Bereich des Glättungszeitfensters von 0 s bis 2,5 s beträgt.

7. Radiofrequenzgenerator für eine Ablation nach einem der Ansprüche 1 bis 6, ferner umfassend einen Schutzmechanismus zum Verhindern einer wiederholenden Ablation, wobei eine Temperatur einer Stelle detektiert wird, welche eine Ablation erfährt, bevor jede Ablation angewendet wird, und falls die Temperatur der Stelle, welche eine Ablation erfährt, höher als 40°C bis 60°C ist, wird eine Ablation nicht gestartet; optional wobei die Temperatur der Stelle detektiert wird, welche eine Ablation erfährt, bevor jede Ablation angewendet wird, und falls die Temperatur der Stelle, welche eine Ablation erfährt, höher als 45°C ist, wird eine Ablation nicht gestartet.

8. Radiofrequenzgenerator für eine Ablation nach Anspruch 1, welcher dazu eingerichtet ist, ein Verfahren 1 zu verwenden, umfassend: Detektieren der Impedanz durch kontinuierliches schwaches Wechselstromsignal und Berechnen der Impedanz durch eine Spannung und einen Strom während einer Radiofrequenzausgabe; und/oder
ein Verfahren 2, umfassend: direktes Detektieren der Impedanz ohne Radiofrequenzausgabe.

9. Radiofrequenzgenerator für eine Ablation nach Anspruch 1, umfassend einen Radiofrequenz-Energieübertragung/Rückkopplung-Steuerungsmechanismus, welcher eingerichtet ist, für: nach der Radiofrequenz-Energieausgabe für 2 bis 4 s, eine Temperatur eines abladierten Gewebes eine festgelegte Temperatur erreicht und für 6 bis 8 s beibehalten wird, ein Übertemperaturalarm ausgegeben wird, wenn die Temperatur des abladierten Gewebes höher als ein Übertemperatur-Schwellenwert ist, und ein Ablationssystem die Radiofrequenz-Energieausgabe automatisch stoppt;
optional wobei die festgelegte Temperatur von 60°C bis 70°C reicht, und wobei der Übertemperatur-Schwellenwert 1°C bis 10°C höher ist als die festgelegte Temperatur;
weiter optional wobei die festgelegte Temperatur 65°C beträgt, und wobei der Übertemperatur-Schwellenwert 3°C höher ist als die festgelegte Temperatur.

10. Radiofrequenzgenerator für eine Ablation nach Anspruch 1, welcher eine Ausgestaltung multipler zentraler Steuerungseinheiten, dualer Temperaturschaltkreise und dualer Spannungs- und Stromschaltkreise annimmt;
oder
wobei der Radiofrequenzgenerator für eine Ablation mit einer Datenübertragungsschnittstelle bereitgestellt ist und dazu in der Lage ist, extern mit einem Computer verbunden zu sein, um in Echtzeit Informationen über verschiedene Parameter zu erhalten; oder
wobei der Radiofrequenzgenerator für eine Ablation mit einem Touch-Anzeigebildschirm zum Anzeigen eines Elektrodenzustands und eines Adhäsion-Impedanzwertes der Elektroden und eines Gewebes bereitgestellt ist, und wobei eine oder eine Mehrzahl von Elektroden dazu in der Lage ist/sind, gesteuert zu werden, um Energie durch Berühren des Touch-Anzeigebildschirms freizugeben.

11. Multi-Elektrode-Ablationsvorrichtung, umfassend den Radiofrequenzgenerator für eine Ablation nach einem der Ansprüche 1 bis 10, eine Elektrodenanordnung (2, 3), einen Führungskatheter (6), einen Griff (17) und ein Verbindungsstück (18),
wobei wenigstens eine Kavität in dem Führungskatheter (6) gebildet ist;
wobei die Elektrodenanordnung (2, 3) an einem vorderen Ende des Führungskatheters (6) angeordnet ist und mit dem Griff (17) verbunden ist, indem ein Schaltkreis durch das Innere des Führungskatheters (6) verläuft, wobei die Elektrodenanordnung (2, 3) eine oder mehrere Elektrodengruppen und eine oder mehrere Detektionsvorrichtungen umfasst, wobei die Elektrodengruppe dazu in der Lage ist, eine elektrische Energie, eine Radiofrequenzenergie, eine Laserenergie, einen fokussierten Ultraschall mit hoher Dichte oder eine niedrige Temperatur für eine Ablation aufzubringen, und wobei die Detektionsvorrichtung dazu eingerichtet ist, eine Temperatur, Impedanz oder Tension zu detektieren;
wobei der Griff (17) mit dem Verbindungsstück (18) und einer oder mehreren Gruppen von Elektrodenanordnungen (2, 3) verbunden ist und eine oder eine Mehrzahl von Operationskomponenten umfasst, welche dazu eingerichtet sind, eine Verengung, Expansion und Energiefreigabe der Elektrodengruppen zu steuern, und welche dazu in der Lage ist, die Elektrodenanordnung (2, 3) zu steuern, um sich aus dem Führungskatheter (6) zu erstrecken oder in diesen zurückzuziehen; und
wobei das Verbindungsstück (18) dazu eingerichtet ist, eine Energie für Elektroden bereitzustellen.

12. Multi-Elektrode-Ablationsvorrichtung nach Anspruch 11, wobei die Detektionsvorrichtung eine Temperatur-Detektionsvorrichtung, eine Impedanz-Detektionsvorrichtung und eine Tension-Detektionsvorrichtung umfasst.

13. Multi-Elektrode-Ablationsvorrichtung nach Anspruch 12, wobei die Elektrodengruppe (2, 3) eine oder eine Mehrzahl von Elektroden umfasst, wobei jede Elektrode durch einen unabhängigen Elektrodenleiter mit dem Griff (17) verbunden ist,
wobei die Elektrodengruppe dazu eingerichtet ist, durch Steuerung der Operationskomponente in einer Korbform, Spiralform oder Ballonform zu expandieren, und wobei, bei Anwesenheit einer Mehrzahl von Elektrodengruppen, die Elektrodengruppen sequenziell in Reihe angeordnet sind;
und wobei je näher die Elektrodengruppen an dem Griff (17) sind, desto größer ein äußerer Durchmesser nach einer Expansion wird, und wobei der äußere Durchmesser 1 bis 20 mm beträgt.

14. Multi-Elektrode-Ablationsvorrichtung nach Anspruch 13, wobei die Elektrodenanordnung (2, 3) ferner einen Zugkraft-Stahldraht (5) umfasst, wobei zwei Enden der Elektroden an dem Zugkraft-Stahldraht (5) fixiert sind, wobei der Zugkraft-Stahldraht (5) durch den Führungskatheter (6) verläuft, um mit dem Griff (17) verbunden zu sein, und wobei der Griff eine Kontraktion und Expansion der Elektrodengruppe durch Ziehen und Freigeben des Zugkraft-Stahldrahts (5) steuert; optional wobei, in Anwesenheit einer Mehrzahl von Elektrodengruppen, eine Schadenverhinderungsstruktur (1) an einem Kopfende der Elektrodengruppe an einem Ende angeordnet ist, welches am entferntesten von dem Griff (17) ist, und wobei die Elektrodengruppen durch Hilfskomponenten verbunden sind, optional wobei ein Drucksensor (20) an dem Zugkraft-Stahldraht (5) angeordnet ist;
oder wobei die Elektrodenanordnung (2, 3) ferner einen Ballon (11) umfasst, welcher zwischen den Elektroden angeordnet ist, wobei der Ballon dazu eingerichtet ist, durch den Führungskatheter (6) durch einen Ballon-Luftdurchgang zu verlaufen, um mit dem Griff (17) verbunden zu sein, und dazu in der Lage ist, durch den Griff (17) mit einem Lufteinlass verbunden zu sein, und wobei die Elektrodengruppe dazu eingerichtet ist, zu expandieren, nachdem der Ballon (11) zum Expandieren aufgeblasen ist; und wobei, in Anwesenheit einer Mehrzahl von Elektrodengruppen, eine Mehrzahl von Ballons (11, 12) sequenziell in Reihe angeordnet sind, und die Mehrzahl von Ballons jeweils durch unabhängige Ballon-Luftdurchgänge (15, 16) mit dem Griff (11, 12) verbunden sind.

15. Multi-Elektrode-Ablationsvorrichtung nach Anspruch 11, wobei je näher der Führungskatheter (6) an dem Griff (7), desto größer die Härte ist, und wobei die Härteverteilung von 90 A zu 80 D einer Shore-Härte reicht; oder
wobei die Operationskomponente des Griffs (17) eine Steuerungsleiterplatte und eine Steuertaste umfasst; wobei die Leiterplatte mit der Elektrodenanordnung (2, 3) und der Steuertaste verbunden ist, und wobei die Steuertaste dazu eingerichtet ist, jeweils verschiedene Komponenten in verschiedenen Elektrodenanordnungen zu steuern, optional wobei die Elektrodengruppe dazu in der Lage ist, eine oder eine Mehrzahl von Elektroden zu steuern, um durch die Steuerung der Operationskomponente des Griffs eine Energie freizugeben; oder
wobei der Radiofrequenzgenerator für eine Ablation dazu in der Lage ist, eine Impedanz oder Tension der Elektrode anzuzeigen und zu indizieren, ob die Elektrodenanordnung gründlich an der trachealen Wand befestigt ist oder nicht: nachdem Elektroden eines bronchialen Radiofrequenzgenerators für eine Ablation an dem Gewebe befestigt sind, indiziert ein Impedanzwert kleiner als oder gleich wie der Schwellenwert eine gründlich Befestigung der Elektrodenanordnung an der bronchialen Wand, optional wobei der Schwellenwert einer Impedanz von 500 Ω bis 1000 Ω reicht, weiter optional wobei der Schwellenwert einer Impedanz 900 Ω beträgt; oder
wobei, gemäß einem Verfahren eines Bestimmens, ob die Elektroden durch den Radiofrequenzgenerator für eine Ablation in gründlichem Kontakt mit der trachealen Wand sind oder nicht, der Radiofrequenzgenerator für eine Ablation dazu in der Lage ist, die Impedanz jeder Elektrode zu messen, und falls die Impedanz konsistent ist, ist der Kontakt zwischen den Elektroden und der trachealen Wand gründlich; und falls der Kontakt zwischen einer bestimmten Elektrode und der trachealen Wand nicht gründlich ist, ist die Impedanz verschieden von der der anderen, welche in gründlichem Kontakt sind.

## Revendications

1. Générateur de radio fréquence pour ablation à utiliser dans un dispositif de transmission d'énergie dans la trachée et les bronches, le générateur étant capable de
générer et commander un courant direct, un courant alternatif et une énergie de radio fréquence,
collecter, traiter et afficher un signal de température, d'impédance ou de tension, et
déterminer l'efficacité d'ablation selon un changement du signal d'impédance ou de tension, et le changement d'impédance est l'un ou plusieurs de
une valeur de chute d'impédance,
un taux de changement d'impédance,
un changement dans le taux de changement d'impédance, ou
un changement d'impédance de la chute à l'élévation,
dans lequel le générateur de radio fréquence pour ablation est configuré pour utiliser un procédé de commande de segmentation afin d'ajuster une puissance de sortie de radio fréquence à travers un système de commande en boucle fermée afin de commander une température d'ablation, et la commande de segmentation comprend :
(1) un stade de chauffage rapide : durant de 0,5 s à 2 s depuis le début de l'ablation, dans lequel une température de point final du stade de chauffage rapide atteint 50 % à 80 % de la température d'ablation ;
(2) un stade de chauffage lent : durant de 0,5 s à 2 s après le stade de chauffage rapide, dans lequel une température de point final du stade de chauffage lent atteint 70 % à 99 % de la température d'ablation, ou est de 0,1°C à 10°C inférieure à la température d'ablation ; et
(3) un stade de maintien de stabilité : maintenant de manière stable la température après le stade de chauffage lent jusqu'à ce que l'ablation s'arrête.

2. Générateur de radio fréquence pour ablation selon la revendication 1, dans lequel l'ablation est déterminée pour être efficace lorsqu'une valeur de chute d'impédance excède 10 Ω à 100 Ω, ou que le taux de changement d'impédance est supérieur à -1 Ω/s à -50 Ω/s, ou que le changement d'impédance passe de chute à élévation ;
éventuellement dans lequel l'ablation est déterminée pour être efficace lorsque la valeur de chute d'impédance excède 20 Ω à 50 Ω, ou que le taux de changement d'impédance est supérieur à -5 Ω/s à - 50 Ω/s, ou que le changement d'impédance passe de chute à élévation.

3. Générateur de radio fréquence pour ablation selon la revendication 1, dans lequel la commande de segmentation comprend : (1) le stade de chauffage rapide : durant 1 s à partir du début de l'ablation, dans lequel une température de point final du stade de chauffage rapide atteint 65 % de la température d'ablation ; (2) le stade de chauffage lent : durant 1 s après le stade de chauffage rapide, dans lequel une température de point final du stade de chauffage lent atteint 90 % de la température d'ablation, ou est de 2°C inférieure à la température d'ablation ; et (3) le stade de maintien de stabilité : maintenant de manière stable la température après le stade de chauffage lent jusqu'à ce que l'ablation s'arrête.

4. Générateur de radio fréquence pour ablation selon la revendication 1, dans lequel le générateur de radio fréquence pour ablation est configuré pour exécuter un lissage dynamique sur la température dans le processus de commande de la température d'ablation, comprenant le calcul de moyenne, la moyenne pondérée ou la moyenne médiane sur une valeur de température d'échantillonnage, le générateur de radio fréquence pour ablation étant guidé pour ajuster la sortie de puissance de radio fréquence en fonction d'une valeur de température obtenue à travers un lissage dynamique, et le changement lissé de la puissance de sortie de radio fréquence dans le procédé d'ablation est ainsi garanti.

5. Générateur de radio fréquence pour ablation selon la revendication 4, dans lequel une limite supérieure d'une valeur seuil du lissage dynamique est de 0,1°C/s à 20°C/s, et une limite inférieure de la valeur seuil est de -0,1°C/s à -20°C/s ; lorsqu'un taux de changement de température est inférieur à la limite inférieure de la valeur seuil, une fenêtre de temps de lissage est prolongée ; lorsque le taux de changement de température est supérieur à la limite supérieure de la valeur seuil, la fenêtre de temps de lissage est raccourcie ; et lorsque le taux de changement de température est compris entre la limite inférieure et la limite supérieure de la valeur seuil, la fenêtre de temps de lissage demeure inchangée.

6. Générateur de radio fréquence pour ablation selon la revendication 5, dans lequel la limite supérieure de la valeur seuil du lissage dynamique est de 5°C/s, et la limite inférieure est de -5°C/s ;
ou dans lequel une plage dynamique de la fenêtre de temps de lissage est de 0 s à 10 s, éventuellement dans lequel une plage dynamique de la fenêtre de temps de lissage est de 0 s à 2,5 s.

7. Générateur de radio fréquence pour ablation selon l'une quelconque des revendications 1 à 6, comprenant en outre un mécanisme de protection pour empêcher la reproduction de l'ablation, dans lequel une température d'un site à ablater est détectée avant que chaque ablation soit appliquée, et si la température du site à ablater est supérieure à 40°C à 60°C, l'ablation n'est pas démarrée ; éventuellement dans lequel la température du site à ablater est détectée avant que chaque ablation soit appliquée, et si la température du site à ablater est supérieure à 45°C, l'ablation n'est pas démarrée.

8. Générateur de radio fréquence pour ablation selon la revendication 1, configuré pour utiliser
un procédé 1 comprenant : la détection de l'impédance par un signal de courant alternatif faible continu, et le calcul de l'impédance à travers une tension et un courant durant une sortie de radio fréquence ; et/ou
un procédé 2 comprenant : la détection directement de l'impédance sans sortie de radio fréquence.

9. Générateur de radio fréquence pour ablation selon la revendication 1, comprenant un mécanisme de commande de transmission/de rétroaction d'énergie de radio fréquence configuré pour : après que la sortie d'énergie de radio fréquence durant 2 à 4 s, une température d'un tissu ablaté atteint une température de consigne et est maintenue durant 6 à 8 s, une alarme de limite de sur température est donnée lorsque la température du tissu ablaté est supérieure à une valeur seuil de limite de sur température, et un système d'ablation arrête automatiquement la sortie d'énergie de radio fréquence ;
éventuellement dans lequel la température de consigne s'étend de 60°C à 70°C, et la valeur seuil de limite de température haute est de 1°C à 10°C supérieure à la température de consigne ;
plus facultativement dans lequel la température de consigne est de 65°C, et la valeur seuil de limite de température haute est de 3°C supérieure à la température de consigne.

10. Générateur de radio fréquence pour ablation selon la revendication 1, adoptant une conception de multiples contrôleurs centraux, circuits de température doubles, et tension double et circuits de courant ; ou
dans lequel le générateur de radio fréquence pour ablation est pourvu d'une interface de transmission de données, et peut être externellement raccordé à un ordinateur pour obtenir des informations de divers paramètres en temps réel ; ou
dans lequel le générateur de radio fréquence pour ablation est pourvu d'un écran d'affichage tactile pour afficher un état des électrodes et une valeur d'impédance d'adhésion des électrodes et d'un tissu, et l'une ou une pluralité d'électrodes peut·peuvent être commandée·s pour libérer de l'énergie en cliquant sur l'écran d'affichage tactile.

11. Dispositif d'ablation à électrodes multiples, comprenant le générateur de radio fréquence pour ablation selon l'une quelconque des revendications 1 à 10, un ensemble d'électrodes (2, 3), un cathéter de guidage (6), une poignée (17) et un connecteur (18),
dans lequel au moins une cavité est formée dans le cathéter de guidage (6) ;
dans lequel l'ensemble d'électrodes (2, 3) est disposé au niveau d'une extrémité avant du cathéter de guidage (6), et est connecté à la poignée (17) en faisant passer un circuit à travers l'intérieur du cathéter de guidage (6), l'ensemble d'électrodes (2, 3) incluant un ou plusieurs groupes d'électrodes et un ou plusieurs dispositifs de détection, le groupe d'électrodes étant capable d'appliquer une énergie électrique, une énergie de radio fréquence, une énergie laser, des ultrasons focalisés à haute densité ou une basse température pour ablation, et le dispositif de détection étant configuré pour détecter une température, une impédance ou une tension ;
dans lequel la poignée (17) est connectée au connecteur (18) et à un ou plusieurs groupes d'ensembles d'électrodes (2, 3), et comprend l'un ou une pluralité de composants opérationnels configurés pour commander la constriction, l'expansion et la libération d'énergie des groupes d'électrodes, et est capable de commander à l'ensemble d'électrodes (2, 3) de se déployer à l'extérieur ou d'effectuer un repli dans le cathéter de guidage (6) ; et
dans lequel le connecteur (18) est configuré pour fournir une énergie aux électrodes.

12. Dispositif d'ablation à électrodes multiples selon la revendication 11, dans lequel le dispositif de détection comprend un dispositif de détection de température, un dispositif de détection d'impédance et un dispositif de détection de tension.

13. Dispositif d'ablation à électrodes multiples selon la revendication 12, dans lequel le groupe d'électrodes (2, 3) comprend l'une ou une pluralité d'électrodes, chaque électrode étant connectée à la poignée (17) à travers un conducteur d'électrode indépendant,
dans lequel le groupe d'électrodes est configuré pour s'expanser en une forme de panier, une forme de spirale ou une forme de ballon sous le contrôle du composant fonctionnel, et en présence d'une pluralité de groupes d'électrodes, les groupes d'électrodes sont séquentiellement agencés en série ;
et dans lequel plus les groupes d'électrodes sont proches de la poignée (17), plus grand se trouve un diamètre externe après expansion, et le diamètre externe est de 1 à 20 mm.

14. Dispositif d'ablation à électrodes multiples selon la revendication 13, dans lequel l'ensemble d'électrodes (2, 3) comprend en outre un fil de traction en acier (5), deux extrémités de l'électrode étant fixées au fil de traction en acier (5), le fil de traction en acier (5) passant à travers le cathéter de guidage (6) pour être raccordé à la poignée (17), et la poignée commandant la contraction et l'expansion du groupe d'électrodes en tirant et en relâchant le fil de traction en acier (5) ; éventuellement dans lequel en présence d'une pluralité de groupes d'électrodes, une structure de prévention de détérioration (1) est disposée à une extrémité de tête du groupe d'électrodes à une extrémité la plus éloignée de la poignée (17), et les groupes d'électrodes sont raccordés à travers des composants de support ; éventuellement dans lequel un capteur de pression (20) est disposé sur le fil de traction en acier (5) ;
ou dans lequel l'ensemble d'électrodes (2, 3) comprend en outre un ballonnet (11) disposé entre les électrodes, dans lequel le ballonnet est configuré pour passer à travers le cathéter de guidage (6) à travers un passage d'air de ballonnet à raccorder à la poignée (17), et est capable d'être raccordé à un appareil d'entrée d'air à travers la poignée (17), et le groupe d'électrodes est configuré pour s'expanser après que le ballonnet (11) soit gonflé pour s'expanser ; et en présence d'une pluralité de groupes d'électrodes, une pluralité de ballonnets (11, 12) sont séquentiellement agencés en série, et la pluralité de ballonnets (11, 12) sont respectivement raccordés à la poignée à travers des passages d'air indépendants (15, 16) de ballonnet.

15. Dispositif d'ablation à électrodes multiples selon la revendication 11, dans lequel plus le cathéter de guidage (6) est proche de la poignée (17), plus grande est la dureté, et la distribution de dureté s'étend de 90 A à 80 D d'une dureté Shore ; ou
dans lequel le composant fonctionnel de la poignée (17) comprend une carte de circuit de commande et un bouton de commande ; le circuit de commande étant raccordé à l'ensemble d'électrodes (2, 3) et au bouton de commande, et le bouton de commande étant configuré pour commander différents composants dans différents ensembles d'électrodes respectivement, éventuellement dans lequel le groupe d'électrodes est capable de commander l'une ou une pluralité d'électrodes pour libérer une énergie sous la commande du composant fonctionnel de la poignée ; ou
dans lequel le générateur de radio fréquence pour ablation est capable d'afficher une impédance ou une tension de l'électrode et d'indiquer si l'ensemble d'électrodes est bien attaché à la paroi trachéenne ou pas : une valeur d'impédance inférieure ou égale à la valeur seuil après que les électrodes d'un générateur de radio fréquence bronchique pour ablation soient fixées au tissu indiquant une bonne fixation de l'ensemble d'électrodes à la paroi bronchique, éventuellement dans lequel la valeur seuil d'impédance s'étend de 500 Ω à 1 000 Ω, plus facultativement dans lequel la valeur seuil d'impédance est de 900 Ω ; ou
dans lequel selon un procédé de détermination du fait que les électrodes se trouvent en bon contact avec la paroi trachéenne ou pas, par le générateur de radio fréquence pour ablation, le générateur de radio fréquence pour ablation est capable de mesurer l'impédance de chaque électrode, et si l'impédance est cohérente, le contact entre les électrodes et la paroi trachéenne est bon ; et si le contact entre une certaine électrode et la paroi trachéenne n'est pas bon, l'impédance est différente de celle d'autres en bon contact.
